# EUROPEAN PATENT SPECIFICATION

(11) **EP 2 638 708 B1**
(45) Date of publication and mention of the grant of the patent: **06.08.2014**
(21) Application number: 10779303.6
(22) Date of filing: 08.11.2010
(51) Int. Cl.: H04R 25/00, A61N 1/36, A61N 1/37

(54) **Hearing instrument and method of operating the same**
Hörgerät und zugehöriges Betriebsverfahren
Instrument auditif et son procédé de fonctionnement

(43) Date of publication of application: 18.09.2013
(73) Proprietor: Advanced Bionics AG, 8712 Stäfa (CH)
(72) Inventor: HAMACHER, Volkmar, 30657 Hannover (DE)
(74) Representative: Schwan Schorer & Partner mbB
(86) International application number: PCT/EP2010/067057
(87) International publication number: WO 2011/015673

(56) References cited:
- WO-A1-01/78449
- WO-A1-2004/008801
- US-A1- 2002 048 382
- US-A1- 2004 190 737
- US-A1- 2006 023 907
- US-A1- 2009 074 216
- US-B1- 6 516 073

## Description

The invention relates to a method of operating a hearing instrument worn at or at least partly in the ear of a user and including a digital audio signal processing unit and an electro-acoustic or electro-mechanical output transducer.

Examples of such type of hearing aids are active middle ear implants and conventional electro-acoustic hearing aids.

Running out of battery power is a permanent issue in particular for users of partially implantable hearing aids, wherein the power required by the implanted part of the hearing aid is supplied by a battery of the external part. While the battery of the external part of the hearing aid in principle can be replaced quiet easily, a spare battery needs to be available and, depending on the situation, the user of the hearing aid may not want to a attract attention when fiddling around with the battery; in addition, during replacement of the battery the hearing aid does not work, so that the user, depending on the degree of his hearing loss, may be more or less deaf. In particular such temporary deafness will be very disturbing in daily life, especially for active people. Battery life time in partially implantable hearing aids typically is on the order of one day.

In principle, users of conventional electro-acoustic hearing aids encounter similar problems, but to a less prominent extent, since ear battery runtimes typically are more than one week and, except for profound losses, the users of electro-acoustic hearing aids typically have a certain level of residual hearing and speech understanding without electronic amplification.

US 7,120,500 B1 relates to a Cochlear implant, wherein, if the battery voltage falls below a first threshold, power consumption is reduced by reducing the stimulation rate applied by the implant as a function of the battery voltage; if the battery voltage falls below a second threshold, the speech processor is shut down and a low voltage alarm is triggered; when the battery voltage raises again above the first threshold value, the stimulation rate returns to the normal programmed rate. If the stimulation rate falls below a minimum rate, a warning beep is issued to indicate to the user that the processor may be shut down shortly. Alternatively, the number of channels processed by the filter and/or the number of channels selected for stimulation may be reduced. At low battery voltage another processing strategy may be selected which copes better with progressive stimulation rate reduction than the normal strategy.

EP 1 727 394 B 1 relates to an electro-acoustic hearing aid, when the hearing aid speaker is temporarily switched on and off in order to prevent the battery voltage to fall below a certain limit at which the digital control circuit of the hearing aid would reset and wherein, if the on/off switching of the speaker occurs too frequently, the gain is progressively reduced until the battery voltage is again above the threshold value. Thus, negative effects of battery voltage drops due to too high currents, as induced by high input/output levels, are to be avoided.

US 2009/0074203 A1 relates to an electro acoustic hearing aid which is connected via an ultra wide band (UWB) link to a belt-won external processing device and to another hearing aid worn at the other ear. The wireless transceiver of the hearing aid may be shut off when it is detected that the power of the hearing aid is low. In additional, the hearing aid is switched to a conventional analog amplifier mode when the hearing aid power is critically low.

US 6,904,156 B1 relates to an electro acoustic hearing aid, wherein the hearing aid audio amplifier is disabled when low battery voltage is sensed. US 2002/0159613 A1 relates to an electro acoustic hearing aid comprising an alarm system providing for an output signal whose amplitude and frequency increases as battery voltage decreases below a predetermined level. Also US 6,310,556 B1 relates to a hearing aid having an alarm system for detecting low battery power condition, wherein in audible warning is generated when the battery output voltage drops below a first threshold voltage. When the battery output voltage drops below a second threshold voltage, the output stage of the hearing aid is shut down. WO 97/01314 A1 relates to a hearing aid comprising a battery monitoring function, wherein an alarm signal is generated when low battery power condition is detected.

US 2009/0074216 A1 relates to a digital hearing aid, wherein, when the battery power is found to be low, a wireless transceiver of the hearing aid is switched off and the hearing aid is switched into an analog amplifier mode in order to save power.

US 2006/0023907 A1 relates a to a hearing aid, wherein a voltage indicator is provided which warns the user in case that the hearing aid battery becomes weaker. If the battery voltage drops below a threshold, a warning signal is fed to the signal processing unitand low frequency signal elements in the output signal are reduced. Further, there may be a gradual reduction of the gain at low frequencies and a gradual increase in the cut-off frequency as a function of the battery voltage.

It is an object of the invention to provide for a hearing instrument worn at or at least partly in the ear of a user and including a digital audio signal processing unit and an electro-acoustic or electro-mechanical output transducer, wherein the time span during which the hearing instrument is able to provide hearing assistance to the user once the battery has entered a stage of low residual battery power is extended. It is a further object to provide for a method of operating such hearing instrument.

According to the invention, these objects are achieved by a method of operating hearing instrument as defined in claim 1 and a hearing instrument as defined in claim 12.

The invention is beneficial in that, by selecting a low power audio signal processing mode having reduced power consumption compared to the standard audio signal processing mode when low battery status has been detected, in addition to providing for an alarm signal to a user, battery life time can be extended while still providing for hearing assistance to the user-although at potentially reduced audio quality - so that the user has more time to replace the battery until the hearing instrument ceases to provide for hearing assistance. Preferably, a warning signal is provided to the user once a low battery status has been detected.

Further preferred embodiments of the invention are defined in the dependent claims.

Hereinafter, examples of the invention will be illustrated by reference to the attached drawings, wherein:
- Fig. 1: is a schematic functional block diagram of an example of a hearing instrument according to the invention;

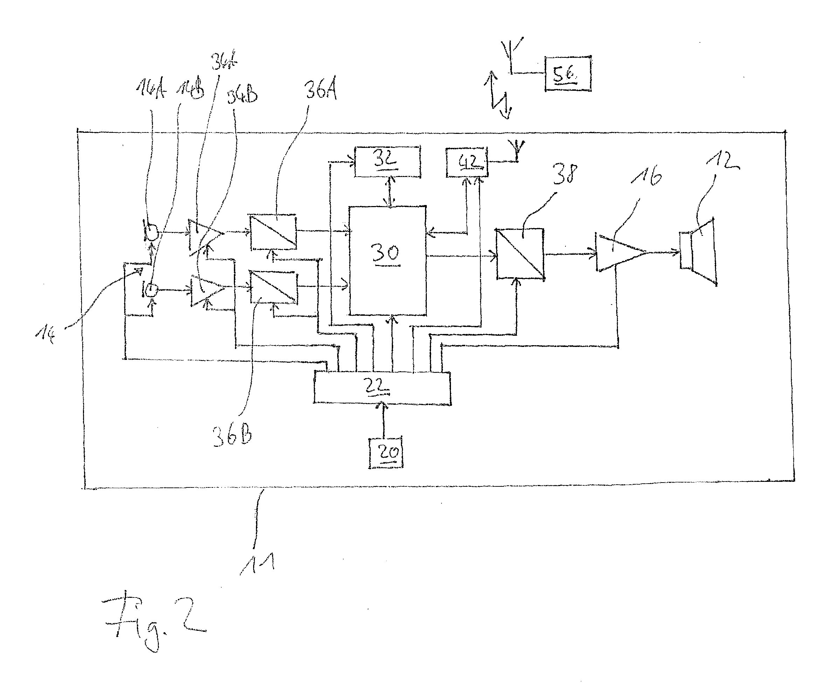

- Fig. 2: is schematic block diagram of the hearing instrument of Fig. 1 wherein some details regarding the electronic components are shown; and
- Fig. 3: is schematic diagram showing an example of how the battery voltage decreases as a function of time in a hearing instrument according to the invention.

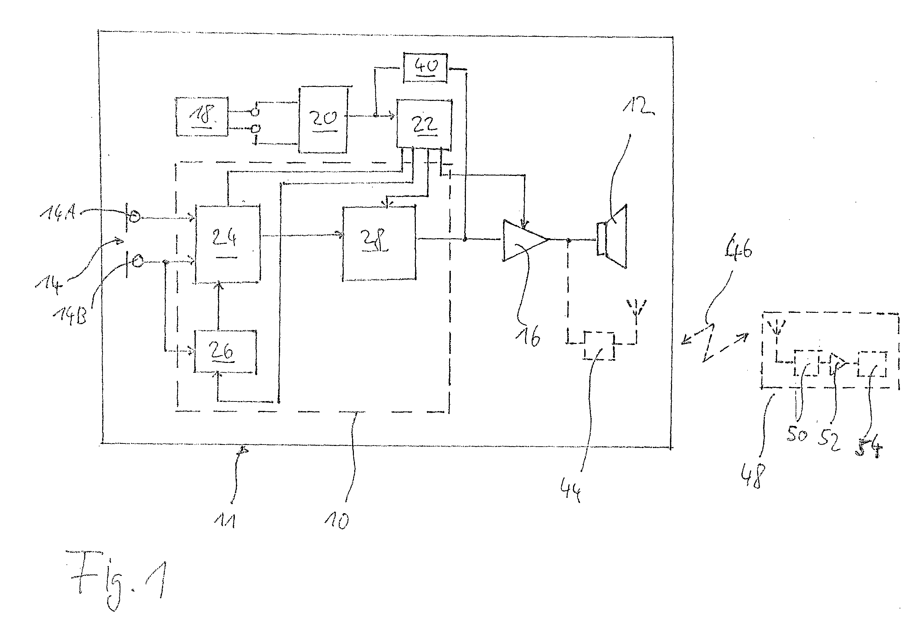

Fig. 1 relates to an example of a hearing instrument 11 to be worn at least partly in the car of a user comprising a digital audio signal processing unit 10 and an electro-acoustic output transducer (speaker/receiver) 12, a microphone arrangement 14 for capturing audio signals from ambient sound, a power amplifier 16, a battery 18, a battery monitoring unit 20 and an audio signal processing mode selection unit 22. The audio signals captured by the microphone arrangement 14 are processed in the audio signal processing unit 10, the processed audio signals are amplified in the power amplifier 16, and the amplified processed audio signals are converted into sound by the receiver 12.

Typically, the audio signal processing unit 10 comprises an acoustic beamformer unit 24, an auditory scene classifier unit 26 and a unit 28 which processes the audio signals provided by the microphone arrangement 14 or the beamformer 24 by applying a frequency and level dependent gain to the audio signals (which are divided, to this end, into a plurality of frequency bands) in order to supply processed audio signals to the power amplifier 16. In order to supply the beam former 24 with the necessary input signals, the microphone arrangement 14 comprises at least two spaced part microphones 14A and 14B. The unit 28 may include processing schemes like feedback canceling, frequency compression, noise reduction and pinna simulation. The specific audio signal processing scheme applied in the beamformer and the unit 28 is selected according to the presently prevailing auditory scene, as detected by the classification unit 26. In practice, the units 24, 26 and 28 are functionally realized by a digital signal processor (DSP) 30 and a memory 32.

The hearing instrument 11 also comprises a pre-amplifier 34A for amplifying the audio signals captured by a microphone 14A and a pre-amplifier 34B for pre-amplifying the audio signals captured by the microphone 14B. The pre-amplified audio signals are supplied to a digital-to-analog converter (DAC) 36A and 36B, respectively, with the corresponding digital signals being supplied is input to the DSP 30. The digital audio output signals of the DSP 30 are supplied to an analog-to-digital converter (ADC) 38 prior being supplied as corresponding analog signals to the amplifier 16.

The hearing instrument 11 also comprises an alarm unit 40 for generating an alarm/warning signal when a low battery status has been detected by the battery monitoring unit 20, which alarm signal is mixed with the audio output signals of the unit 28 in order to indicate to the user that the battery 18 needs to be replaced (typically the alarm signal is supplied only for a certain time period after detection of the low battery status).

Once the monitoring unit 20 has detected a low battery status, namely that the residual energy of the battery is low a pre-defined energy threshold, not only the alarm signal of the alarm unit 40 triggered but also the hearing instrument 11 is made to enter a low power audio signal processing mode having reduced power consumption compared to the standard audio signal processing mode (the hearing instrument 11 is operated the standard audio signal processing mode as long as the monitoring unit 20 detects a high battery status in which the residual energy of the battery 18 is above the pre-defined energy threshold).

Such change into a low power audio signal processing mode is achieved by action of the audio signal processing mode selection unit 22, which may act on the following system components: the DSP 30, the memory 32, the microphones 14A, 14B, the microphone pre-amplifiers 34A and 34B, the DACs 36A, 36B, the ADC 38 and the power amplifier 16. For example, the unit 22 may serve to reduce power consumption of the audio signal pre-amplifiers 34A, 34B, the DACs 36A, 36B and/or the ADC 38 by reducing the dynamic range thereof.

In addition, the unit 22 may serve to switch off, at least on a regular temporary base for at least reducing the duty cycle thereof, parts of the DSP 30 and/or the memory 32. When reducing the power consumption of the DSP 30, entire processing cores (if there is a multicore architecture) or parts thereof may be switched off. The unit 22 also may act on at least one of the microphones 14A, 14B to switch off the respective microphone.

The hearing instrument 11 may comprise a unit 42 for reception and/or detection of incoming wireless audio signals or control data via a wireless link from an external device 56, such a wireless remote microphone or another hearing instrument 11. The unit 42 comprises a transceiver (in case of a bidirectional - usually digital - link) or a receiver (in case of an unidirectional - usually analog - link). Incoming signals detected by the receiver / transceiver unit 42 may include baseband or RF (radio frequency) signals including audio data and/or control data. The unit 42 may be formed by a T-coil unit or an FM (frequency modulation) receiver. The data may be received via a standard data network, such as a Bluetooth or Zigbee network.

The control unit 22 may act to completely switch off such transceiver/receiver unit 42 or to make it reduce the data exchange rate via the wireless link. In any case, the transceiver unit 42 may be switched off by the control unit 22 at least on a regular temporary base for at least reducing the duty cycle thereof.

Preferably, when in the low power audio signal processing mode a certain circuitry is switched off by the unit 22, the clock input of said circuitry and/or the supply voltage to said circuitry may be switched off.

More on a functional basis, the unit 22 may change the audio signal processing in the unit 28 in a manner that the gain at pre-defined frequencies compared to the gain at other frequencies less relevant for speech intelligibility may be selectively reduced. In particular, the gain at frequencies below 0.5 kHz and/or above 4 kHz may be reduced relative to the gain at frequencies between 0.5 and 4 kHz. In particular, the frequencies at which the gain is reduced in the low power audio signal processing mode and/or the extent to which the gain is reduced at said frequencies in the low power audio signal processing mode may be selected based on a spectral power consumption profile and the articulation index.

Also, the following audio signal processing functions of the audio signal processing unit 10 may be switched off for at least part of the audio frequencies: feedback canceling, auditory scene classification, frequency compression, noise reduction, pinna simulation and acoustic beamforming. For example, when switching off the acoustic beamforming function, the beam former unit 24 and one of the two audio signal input channels including the respective microphone 14A, 14B, the pre-amplifier 34A, 34B and the ADC 36A, 36B may be switched off. Alternatively, the acoustic beam forming function may switched off only partially by reducing the number of frequency bands.

In the low power audio signal processing mode also the maximum loudness the hearing instrument 11 may achieve may be reduced.

At least part of the power consumption savings achieved by switching off feedback canceling, auditory scene classification, frequency compression, noise reduction, pinna simulation and acoustic beamforming is achieved by the correspondingly reduced data processing requirements, whereby power consumption of the DSP 30 is reduced.

In addition to the above described low power audio signal processing mode a very low power mode is implemented, wherein, when the monitoring unit 20 determines that the residual battery energy is below a second threshold value lower than the energy threshold value value of the low battery status (such battery condition corresponds to a very low battery status of the hearing instrument 11), a speech monitoring function is activated by the unit 22 for determining from the audio signals captured by the microphone arrangement 14 whether speech signals are present at the microphone arrangement 14, and wherein all functions of the hearing instrument 11 except for a speech monitoring function are disabled as long as no speech signals are detected (for the speech monitoring function it is sufficient that only one of the microphones 14A, 14B is active). Thereby the hearing instrument 11 enters a kind of the power saving "sleep mode" as long as no speech is detected. When the battery has such very low battery status, part of the hearing instrument 11 functions are reactivated as long as speech signals are detected; in particular, the DSP 30, especially its output stage, may be reactivated.

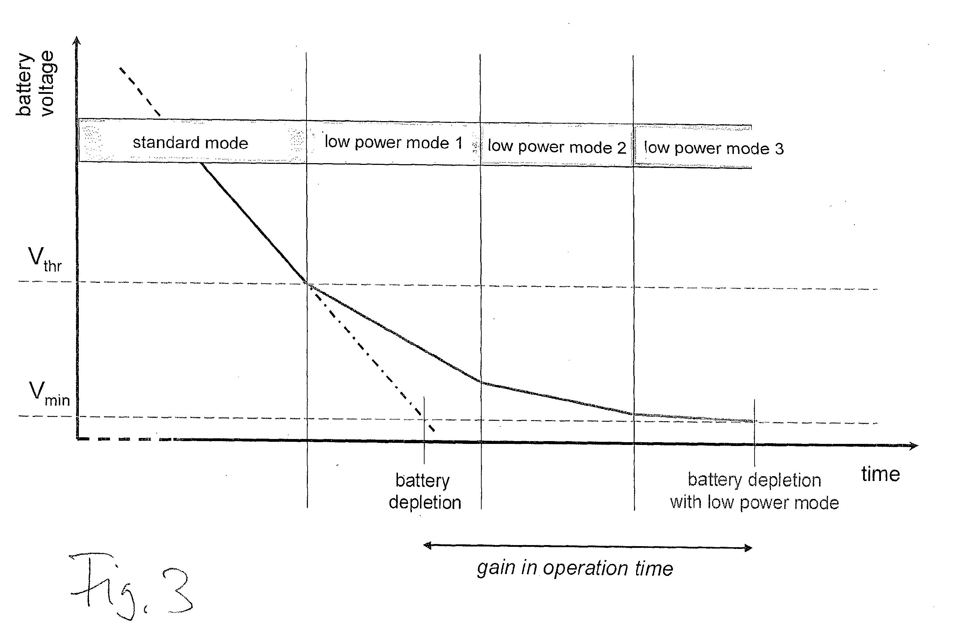

A plurality of different low power audio signal processing modes may be implemented which are selected subsequently by the unit 22 as a function of the residual energy level (voltage) of the battery 18 as detected by the unit 20. In particular, the lower the detected residual energy of the battery 18 is, the more pronounced the reduction in power consumption (and the corresponding reduction of audio performance) has to be. In Fig. 3 an example is shown of how the battery voltage descreases with time when three different low power audio signal processing modes 1 to 3 are employed subsequently, wherein the power consumption - and the residual functionality - of the hearing instrument is subsequently reduced when advancing from the standard mode (which is left when the battery voltage reaches a threashold Vₜₕᵣ) to the low power modes 1 to 3 (which are triggered by respective threashold voltages). Thus, the minimum operating voltage Vₘᵢₙ of the battery is reached later compared to the case without low power modes (dashed line in Fig. 3).

The respective measures taken in the respective low power audio signal processing mode may be defined individually in order to provide the optimal trade-off between acceptable sound quality degradations and the achievable operation time extension for the individual user.

The present invention not only may be applied to hearing instruments having an electro-acoustic output transducer, but rather also may be applied to hearing instruments comprising an active middle ear implant. Such a hearing instrument is schematically indicated in Fig. 1 in dashed lines, wherein the processed audio signals are supplied to a transmission unit 44 for transmitting the audio signals via a transcutaneous wireless link 46 to an implanted arrangement 48 comprising a receiver 50, and amplifier 52 and an electro mechanical output transducer 54 which may be mechanically coupled to the ear drum, the ossicular chain or the cochlea.

## Claims

1. A method of operating a hearing instrument (11) worn at or at least partly in the ear of a user and including a digital audio signal processing unit (10, 24, 28) and an electro-acoustic (12) or electro-mechanical output transducer (54) for stimulating the user's hearing according audio signals processed by the digital audio signal processing unit, comprising:
monitoring the residual energy of a battery (18) of the hearing instrument in order to detect a low battery status of the hearing instrument if the residual energy is below a predefined energy threshold and a high battery status of the hearing instrument if the residual energy is above said energy threshold;
selecting, as long as a high battery status is detected, a standard audio signal processing mode; and,
once a low battery status has been detected, selecting a low power audio signal processing mode having reduced power consumption compared to the standard audio signal processing mode in order to extend battery lifetime, wherein the low power audio signal processing mode includes at least one of the following changes compared to the standard audio signal processing mode:
selectively reducing the gain at predefined frequencies compared to the gain at other frequencies less relevant for speech intelligibility;
switching-off at least one of the following audio signal processing functions for at least part of the audio frequencies: feedback canceling, auditory scene classification,
frequency compression, noise reduction, pinna simulation, and acoustic beamforming;
switching off, at least on a regular temporary base for at least reducing the duty cycle thereof, parts of a processor (30) and/or a memory (32) of the digital audio signal processing unit;
reducing power consumption of an audio signal preamplifier (34A, 34B), an audio signal analog-to-digital converter (36A, 36B) and/or an audio signal digital-to-analog converter (38) by reducing the dynamic range thereof
**characterized in that** in monitoring the residual energy of the battery of the hearing instrument (11) a very low battery status of the hearing instrument is detected if the residual energy is below a second threshold lower than the energy threshold, and wherein, once a very low battery status of the hearing instrument is detected, a speech monitoring function is activated for determining from the audio signals captured by a microphone (14A, 14B) of the hearing instrument whether speech signals are present at the microphone, wherein all functions of the hearing instrument except for speech monitoring function are disabled as long as no speech signals are detected, and wherein part of the hearing instrument functions are reactivated as long as speech signals are detected.

2. The method of claim 1, wherein in the low power audio signal processing mode the gain at frequencies below 0.5 kHz and/or above 4 kHz is reduced relative to the gain at frequencies between 0.5 and 4 kHz.

3. The method of one of the preceding claims, wherein, when switching off the acoustic beamforming function, at least one of the audio signal channels including the respective microphone (14A, 14B), the preamplifier 34A, 34B) and the analog-to-digital converter (36A, 36B) is switched off.

4. The method of one of the preceding claims, wherein, when switching off the acoustic beamforming function, the number of frequency bands is reduced.

5. The method of one of the preceding claims, wherein in the low power audio signal processing mode the maximum loudness of the hearing instrument output is reduced.

6. The method of one of the preceding claims, wherein in the low power audio signal processing mode circuitry (42) for reception and/or detection of incoming wireless audio signals or control data from an external device (56) is switched off at least on a regular temporary base for at least reducing the duty cycle thereof.

7. The method of one of the preceding claims, wherein in the low power audio signal processing mode a binaural wireless data link to another hearing instrument (56) is switched off or the data exchange rate via such binaural wireless data link is reduced.

8. The method of one of the preceding claims, wherein when in the low power audio signal processing mode a certain circuitry (30, 32) is switched off, the clock input of that circuitry and/or the supply voltage to that circuitry is switched off.

9. The method of one of the preceding claims, wherein a warning signal is provided to the user once a low battery status has been detected.

10. The method of one of the preceding claims, wherein the hearing instrument (11) comprises an electro-acoustic transducer (12) for vibrating the user's ear drum.

11. The method of one of claims 1 to 9, wherein the hearing instrument (11) comprises an active middle ear implant (48, 50, 52, 54).

12. A hearing instrument to be worn at or at least partly in the ear of a user and including a digital audio signal processing unit (10, 24, 28) and an electro-acoustic (12) or electro-mechanical output transducer (54) for stimulating the user's hearing according audio signals processed by the digital audio signal processing unit, comprising:
means (20) for monitoring the residual energy of a battery (18) of the hearing instrument (11) in order to detect a low battery status of the hearing instrument if the residual energy is below a predefined energy threshold and a high battery status of the hearing instrument if the residual energy is above said energy threshold;
means (22) for selecting, as long as a high battery status is detected, a standard audio signal processing mode of the audio signal processing unit and for selecting, once a low battery status has been detected, a low power audio signal processing mode of the audio signal processing unit having reduced power consumption compared to the standard audio signal processing mode in order to extend battery lifetime, wherein the low power audio signal processing mode includes at least one of the following changes compared to the standard audio signal processing mode:
selectively reducing the gain at predefined frequencies compared to the gain at other frequencies less relevant for speech intelligibility;
switching-off at least one of the following audio signal processing functions for at least part of the audio frequencies: feedback canceling, auditory scene classification, frequency compression, noise reduction, pinna simulation, and acoustic beamforming;
switching off, at least on a regular temporary base for at least reducing the duty cycle thereof, parts of the processor (30) and/or the memory (32) of the digital audio signal unit;
reducing power consumption of an audio signal preamplifier (34A, 34B), an audio signal analog-to-digital converter (36A, 36B) and/or an audio signal digital-to-analog converter (38) by reducing the dynamic range thereof; and
means (12, 16, 40, 54) for providing, once a low battery status has been detected, a warning signal to the user,
**characterized in that** the monitoring means and the selecting means are designed such that in monitoring the residual energy of the battery of the hearing instrument (11) a very low battery status of the hearing instrument is detected if the residual energy is below a second threshold lower than the energy threshold, and wherein, once a very low battery status of the hearing instrument is detected, a speech monitoring function is activated for determining from the audio signals captured by a microphone (14A, 14B) of the hearing instrument whether speech signals are present at the microphone, wherein all functions of the hearing instrument except for speech monitoring function are disabled as long as no speech signals are detected, and wherein part of the hearing instrument functions are reactivated as long as speech signals are detected.

## Patentansprüche

1. Verfahren zum Betreiben einer am oder mindestens teilweise im Ohr eines Nutzers zu tragenden Hörvorrichtung (11), welche eine digitale Audiosignalverarbeitungseinheit (10, 24, 28) und einen elektroakustischen (12) oder elektromechanischen Ausgangswandler (54) zum Stimulieren des Gehörs des Nutzers gemäß von der digitalen Audiosignalverarbeitungseinheit verarbeiteten Audiosignalen, wobei:
die Restenergie einer Batterie (18) der Hörvorrichtung überwacht wird, um einen niedrigen Batteriestatus der Hörvorrichtung zu erfassen, falls die Restenergie unterhalb eines vorbestimmten Energieschwellwerts liegt, und einen hohen Batteriestatus der Hörvorrichtung zu erfassen, falls die Restenergie oberhalb des Energieschwellwerts liegt;
solange ein hoher Batteriestatus erfasst wird, ein Standardaudiosignalverarbeitungsmodus ausgewählt wird; und
sobald ein niedriger Batteriestatus erfasst wurde, ein Niedrigleistungsaudiosignalverarbeitungsmodus ausgewählt wird, der einen verringerten Energieverbrauch im Vergleich zu dem Standardaudiosignalverarbeitungsmodus aufweist, um die Batterielebensdauer zu verlängern, wobei der Niedrigleistungsaudiosignalverarbeitungsmodus mindestens eine der folgenden Veränderungen im Vergleich zum Standardaudiosignalverarbeitungsmodus aufweist:
selektives Verringern der Verstärkung bei vorbestimmten Frequenzen im Vergleich zu der Verstärkung bei anderen Frequenzen, die weniger wichtig für die Sprachverständigkeit sind;
Abschalten mindestens einer der folgenden Audiosignalverarbeitungsfunktionen für mindestens einen Teil der Audiofrequenzen: Rückkopplungsaufhebung, Hörumgebungsklassifizierung, Frequenzkompression, Rauschverringerung, Pinnasimulation, sowie Bildung einer akustischen Richtcharakteristik;
Ausschalten von Teilen eines Prozessors (30) und/oder eines Speichers (32) der digitalen Audiosignalverarbeitungseinheit mindestens auf einer regelmäßigen zeitlichen Basis, um dessen bzw. deren Einschaltdauer mindestens zu verringern;
Verringern des Energieverbrauchs eines Audiosignalvorverstärkers (34A, 34B), eines Audiosignal-Analog-Digital-Wandlers (36A, 36B) und/oder eines Audiosignal-Digital-Analog-Wandlers (38) mittels Verringerung dessen bzw. deren Dynamikbereichs,
**dadurch gekennzeichnet, dass** beim Überwachen der Restenergie der Batterie der Hörvorrichtung (11) ein sehr niedriger Batteriestatus der Hörvorrichtung erfasst wird, falls die Restenergie unterhalb eines zweiten Schwellwerts liegt, der niedriger liegt als der Energieschwellwert, wobei, sobald ein sehr niedriger Batteriestatus der Vorrichtung erfasst wird, eine Sprachüberwachungsfunktion aktiviert wird, um aus den mittels eines Mikrofons (14A, 14B) der Hörvorrichtung aufgefangenen Audiosignalen zu bestimmen, ob an dem Mikrofon Sprachsignale vorliegen, wobei alle Funktionen der Hörvorrichtung, mit Ausnahme der Sprachüberwachungsfunktion, deaktiviert werden, solange keine Sprachsignale erfasst werden, und wobei ein Teil der Hörinstrumentfunktionen reaktiviert wird, solange Sprachsignale erfasst werden.

2. Verfahren gemäß Anspruch 1, wobei in dem Niedrigenergieaudiosignalverarbeitungsmodus die Verstärkung bei Frequenzen unterhalb 0,5 kHz und/oder oberhalb 4 kHz relativ zu der Verstärkung bei Frequenzen zwischen 0,5 und 4 kHz verringert wird.

3. Verfahren gemäß einem der vorhergehenden Ansprüche, wobei, wenn die akustische Richtcharakteristikbildungsfunktion abgeschaltet wird, mindestens einer der Audiosignalkanäle, einschließlich des entsprechenden Mikrofons (14A, 14B), des Vorverstärkers (34A, 34B) und des Analog-Digital-Wandlers (36A, 36B), ausgeschaltet wird.

4. Verfahren gemäß einem der vorhergehenden Ansprüche, wobei, wenn die akustische Richtcharakteristikbildungsfunktion ausgeschaltet wird, die Zahl von Frequenzbändern verringert wird.

5. Verfahren gemäß einem der vorhergehenden Ansprüche, wobei in dem Niedrigleistungsaudiosignalverarbeitungsmodus die maximale Lautheit der Hörvorrichtungsausgabe verringert wird.

6. Verfahren gemäß einem der vorhergehenden Ansprüche, wobei in dem Niedrigleistungsaudiosignalverarbeitungsmodus eine Schaltung (42) zum Empfangen und/oder Erfassen von eingehenden drahtlosen Audiosignalen oder Steuerdaten von einer externen Vorrichtung (56) mindestens auf einer regelmäßigen zeitlichen Basis abgeschaltet wird, um deren Einschaltdauer mindestens zu verringern.

7. Verfahren gemäß einem der vorhergehenden Ansprüche, wobei in dem Niedrigenergieaudiosignalverarbeitungsmodus eine binaurale drahtlose Datenverbindung zu einer anderen Hörvorrichtung (56) ausgeschaltet wird oder die Datenaustauschrate über eine solche binaurale drahtlose Datenverbindung verringert wird.

8. Verfahren gemäß einem der vorhergehenden Ansprüche, wobei, wenn in dem Niedrigenergieaudiosignalverarbeitungsmodus eine bestimmte Schaltung (30, 32) ausgeschaltet wird, der Takteingang der Schaltung und/oder die Versorgungsspannung zu dieser Schaltung ausgeschaltet wird bzw. werden.

9. Verfahren gemäß einem der vorhergehenden Ansprüche, wobei dem Nutzer ein Warnsignal zugeführt wird, sobald ein niedriger Batteriestatus erfasst wird.

10. Verfahren gemäß einem der vorhergehenden Ansprüche, wobei die Hörvorrichtung (11) einen elektroakustischen Wandler (12) zur Schwingungsanregung des Trommelfells des Nutzers aufweist.

11. Verfahren gemäß einem der Ansprüche 1 bis 9, wobei die Hörvorrichtung (11) ein aktives Mittelohrimplantat (48, 50, 52, 54) aufweist.

12. Am oder mindestens teilweise im Ohr eines Nutzers zu tragende Hörvorrichtung, die eine digitale Audiosignalverarbeitungseinheit (10, 24, 28) und einen elektroakustischen (12) oder elektromechanischen Ausgangswandler (54) zum Stimulieren des Gehörs des Nutzers gemäß von der digitalen Audiosignalverarbeitungseinheit verarbeiteten Audiosignalen aufweist, mit:
Mitteln (20) zum Überwachen der Restenergie einer Batterie (18) der Hörvorrichtung (11), um einen niedrigen Batteriestatus der Hörvorrichtung zu erfassen, falls die Restenergie unterhalb eines vorbestimmten Energieschwellwerts liegt, und einen hohen Batteriestatus der Hörvorrichtung zu erfassen, falls die Restenergie oberhalb des Energieschwellwerts liegt;
Mitteln (22) zum Auswählen eines Standardaudiosignalverarbeitungsmodus der Audiosignalverarbeitungseinheit, solange ein hoher Batteriestatus erfasst wird, und zum Auswählen eines Niedrigenergieaudiosignalverarbeitungsmodus der Audiosignalverarbeitungseinheit, sobald ein niedriger Batteriestatus erfasst wird, wobei der Niedrigenergieaudiosignalverarbeitungsmodus einen verminderten Energieverbrauch im Vergleich zum Standardaudiosignalverarbeitungsmodus zwecks Verlängerung der Batterielebensdauer aufweist, wobei der Niedrigenergieaudiosignalverarbeitungsmodus mindestens eine der folgenden Änderungen im Vergleich zum Standardaudiosignalverarbeitungsmodus aufweist:
selektives Verringern der Verstärkung bei vorbestimmten Frequenzen im Vergleich zur Verstärkung bei anderen Frequenzen, die weniger wichtig für das Sprachverständnis sind;
Abschalten mindestens einer der folgenden Audiosignalverarbeitungsfunktionen für mindestens einen Teil der Audiofrequenzen: Rückkopplungsaufhebung, Hörumgebungsklassifizierung, Frequenzkompression, Rauschverringerung, Pinnasimulation und Bildung einer akustischen Richtcharakteristik;
Abschalten von Teilen des Prozessors (30) und/oder des Speichers (32) der Audiosignalverarbeitungseinheit mindestens auf einer regelmäßigen zeitlichen Basis, um dessen bzw. deren Einschaltdauer mindestens zu verringern;
Verringerung des Energieverbrauchs eines Audiosignalvorverstärkers (34A, 34B), eines Audiosignal-Analog-Digital-Wandlers (34A, 34B) und/oder eines Audiosignal-Digital-Analog-Wandlers (38) mittels Verringerung dessen bzw. deren Dynamikbereichs; und
Mittel (12, 16, 40, 54) zum Darbieten eines Warnsignals an den Nutzer, sobald ein niedriger Batteriestatus erfasst wurde,
**dadurch gekennzeichnet, dass** die Überwachungsmittel und die Auswahlmittel so ausgelegt sind, dass beim Überwachen der Restenergie der Batterie der Hörvorrichtung (11) ein sehr niedriger Batteriestatus der Hörvorrichtung erfasst wird, falls die Restenergie unterhalb eines zweiten Schwellwerts liegt, der niedriger liegt als der Energieschwellwert, und wobei, sobald ein sehr niedriger Batteriestatus der Hörvorrichtung erfasst wird, eine Sprachüberwachungsfunktion aktiviert wird, um aus den von einem Mikrofon (14A, 14B) der Hörvorrichtung aufgefangenen Audiosignalen festzustellen, ob Sprachsignale an dem Mikrofon vorhanden sind, wobei alle Funktionen der Hörvorrichtung, mit Ausnahme der Sprachüberwachungsfunktion, deaktiviert werden, solange keine Sprachsignale erfasst werden, und wobei ein Teil der Hörvorrichtungsfunktionen reaktiviert wird, solange Sprachsignale erfasst werden.

## Revendications

1. Procédé d'actionnement d'un instrument d'audition (11) porté dans l'oreille d'un utilisateur ou au moins partiellement dans celle-ci et comprenant une unité de traitement du signal audio numérique (10, 24, 28) et un transducteur de sortie électro-acoustique (12) ou électro-mécanique (54) pour stimuler l'audition de l'utilisateur en fonction de signaux audio traités par l'unité de traitement du signal audio numérique, comprenant :
le contrôle de l'énergie résiduelle d'une pile ou d'une batterie (18) de l'instrument d'audition de façon à détecter un état de pile ou de batterie faible de l'instrument d'audition si l'énergie résiduelle est inférieure à un seuil d'énergie prédéfini et un état de pile ou de batterie élevé de l'instrument d'audition si l'énergie résiduelle est supérieure audit seuil d'énergie ;
la sélection, tant que l'état de pile ou de batterie élevé est détecté, d'un mode de traitement du signal audio standard ; et
une fois qu'un état de pile ou de batterie faible a été détecté, la sélection d'un mode de traitement du signal audio de faible puissance ayant une consommation d'énergie réduite par rapport au mode de traitement du signal audio standard afin de prolonger une durée de vie de pile ou de batterie, le mode de traitement du signal audio de faible puissance comprenant au moins l'un des changements suivants par rapport au mode de traitement du signal audio standard :
la réduction sélective du gain à des fréquences prédéfinies par rapport au gain à d'autres fréquences moins pertinentes pour l'intelligibilité de la parole ;
l'interruption d'au moins l'une des fonctions de traitement du signal audio suivantes pour au moins une partie des fréquences audio : l'élimination de la rétroaction, la classification de scène auditive, la compression de fréquence, la réduction du bruit, la simulation du pavillon, et la formation de faisceau acoustique ;
la mise hors service, au moins sur une base temporaire régulière au moins pour réduire le rapport cyclique de celle-ci, de parties d'un processeur (30) et/ou d'une mémoire (32) de l'unité de traitement du signal audio numérique ;
la réduction de la consommation d'énergie d'un préamplificateur de signal audio (34A, 34B), d'un convertisseur analogique/numérique de signal audio (36A, 36B) et/ou d'un convertisseur numérique/analogique de signal audio (38) par réduction de la plage dynamique de ceux-ci,
**caractérisé en ce que**, lors du contrôle de l'énergie résiduelle de la pile ou de la batterie de l'instrument d'audition (11), un état de pile ou de batterie très faible de l'instrument d'audition est détecté si l'énergie résiduelle est inférieure à un deuxième seuil inférieur au seuil d'énergie, et dans lequel, une fois qu'un état de pile ou de batterie très faible de l'instrument d'audition est détecté, une fonction de contrôle de la parole est activée pour déterminer à partir des signaux audio capturés par un microphone (14A, 14B) de l'instrument d'audition si des signaux de parole sont ou non présents au niveau du microphone, dans lequel toutes les fonctions de l'instrument d'audition, à l'exception de la fonction de contrôle de la parole, sont désactivées tant qu'aucun signaux de parole ne sont détectés, et dans lequel une partie des fonctions de l'instrument d'audition sont réactivées tant que des signaux de parole sont détectés.

2. Procédé selon la revendication 1, dans lequel, dans le mode de traitement du signal audio de faible puissance, le gain à des fréquences inférieures à 0,5 kHz et/ou supérieures à 4 kHz est réduit par rapport au gain à des fréquences entre 0,5 et 4kHz.

3. Procédé selon l'une des revendications précédentes, dans lequel, lors de l'interruption de la fonction de formation de faisceau acoustique, au moins l'un des canaux de signal audio, comprenant le microphone (14A, 14B), le préamplificateur (34A, 34B) et le convertisseur analogique/numérique (36A, 36B) respectifs, est mis hors service.

4. Procédé selon l'une des revendications précédentes, dans lequel, lors de l'interruption de la fonction de formation de faisceau acoustique, le nombre de bandes de fréquence est réduit.

5. Procédé selon l'une des revendications précédentes, dans lequel, dans le mode de traitement du signal audio de faible puissance, le volume maximal de la sortie de l'instrument d'audition est réduit.

6. Procédé selon l'une des revendications précédentes, dans lequel, dans le mode de traitement du signal audio de faible puissance, des circuits (42) pour la réception et/ou la détection de données de commande ou de signaux audio sans fils entrants venant d'un dispositif extérieur (56) sont mis hors service au moins sur une base temporaire régulière afin de réduire au moins le rapport cyclique de ceux-ci.

7. Procédé selon l'une des revendications précédentes, dans lequel, dans le mode de traitement du signal audio de faible puissance, une liaison de données sans fil biauriculaire avec un autre instrument d'audition (56) est interrompue, ou le débit d'échange de données par l'intermédiaire de cette liaison de données sans fil biauriculaire est réduit.

8. Procédé selon l'une des revendications précédentes, dans lequel, dans le mode de traitement du signal audio de faible puissance, un certain circuit (30, 32) est mis hors service, l'entrée d'horloge de ce circuit et/ou la tension d'alimentation de ce circuit est interrompue.

9. Procédé selon l'une des revendications précédentes, dans lequel un signal d'alarme est délivré à l'utilisateur une fois qu'un état de pile ou de batterie faible a été détecté.

10. Procédé selon l'une des revendications précédentes, dans lequel l'instrument d'audition (11) comprend un transducteur électro-acoustique (12) pour faire vibrer le tympan de l'utilisateur.

11. Procédé selon l'une des revendications 1 à 9, dans lequel l'instrument d'audition (11) comprend un implant d'oreille moyenne actif (48, 50, 52, 54).

12. Instrument d'audition destiné à être porté dans l'oreille d'un utilisateur ou au moins partiellement dans celle-ci et comprenant une unité de traitement du signal audio numérique (10, 24, 28) et un transducteur de sortie électro-acoustique (12) ou électro-mécanique (54) pour stimuler l'audition de l'utilisateur en fonction de signaux audio traités par l'unité de traitement du signal audio numérique, comprenant :
des moyens (20) pour contrôler l'énergie résiduelle d'une pile ou d'une batterie (18) de l'instrument d'audition (11) de façon à détecter un état de pile ou de batterie faible de l'instrument d'audition si l'énergie résiduelle est inférieure à un seuil d'énergie prédéfini et un état de pile ou de batterie élevé de l'instrument d'audition si l'énergie résiduelle est supérieure audit seuil d'énergie ;
des moyens (22) pour sélectionner, tant que l'état de pile ou de batterie élevé est détecté, un mode de traitement du signal audio standard de l'unité de traitement du signal audio et pour sélectionner, une fois qu'un état de pile ou de batterie faible a été détecté, un mode de traitement du signal audio de faible puissance de l'unité de traitement du signal audio ayant une consommation d'énergie réduite par rapport au mode de traitement du signal audio standard afin de prolonger une durée de vie de pile ou de batterie, le mode de traitement du signal audio de faible puissance comprenant au moins l'un des changements suivants par rapport au mode de traitement du signal audio standard :
la réduction sélective du gain à des fréquences prédéfinies par rapport au gain à d'autres fréquences moins pertinentes pour l'intelligibilité de la parole ;
l'interruption d'au moins l'une des fonctions de traitement du signal audio suivantes pour au moins une partie des fréquences audio : l'élimination de la rétroaction, la classification de scène auditive, la compression de fréquence, la réduction du bruit, la simulation du pavillon, et la formation de faisceau acoustique ;
la mise hors service, au moins sur une base temporaire régulière au moins pour réduire le rapport cyclique de celle-ci, de parties du processeur (30) et/ou de la mémoire (32) de l'unité de traitement du signal audio numérique ;
la réduction de la consommation d'énergie d'un préamplificateur de signal audio (34A, 34B), d'un convertisseur analogique/numérique de signal audio (36A, 36B) et/ou d'un convertisseur numérique/analogique de signal audio (38) par réduction de la plage dynamique de ceux-ci ; et
des moyens (12, 16, 40, 54) pour délivrer, une fois qu'un état de pile ou de batterie faible a été détecté, un signal d'alarme à l'utilisateur,
**caractérisé en ce que** les moyens de contrôle et les moyens de sélection sont conçus de telle sorte que, lors du contrôle de l'énergie résiduelle de la pile ou de la batterie de l'instrument d'audition (11), un état de pile ou de batterie très faible de l'instrument d'audition est détecté si l'énergie résiduelle est inférieure à un deuxième seuil inférieur au seuil d'énergie, et dans lequel, une fois qu'un état de pile ou de batterie très faible de l'instrument d'audition est détecté, une fonction de contrôle de la parole est activée pour déterminer à partir des signaux audio capturés par un microphone (14A, 14B) de l'instrument d'audition si des signaux de parole sont ou non présents au niveau du microphone, dans lequel toutes les fonctions de l'instrument d'audition, à l'exception de la fonction de contrôle de la parole, sont désactivées tant qu'aucun signaux de parole ne sont détectés, et dans lequel une partie des fonctions de l'instrument d'audition sont réactivées tant que des signaux de parole sont détectés.
